# EUROPÄISCHE PATENTSCHRIFT

(11) **EP 1 189 582 B1**
(45) Veröffentlichungstag und Bekanntmachung des Hinweises auf die Patenterteilung: **08.03.2006**
(21) Anmeldenummer: 00951293.0
(22) Anmeldetag: 23.06.2000
(51) Int. Cl.: A61K 6/06, A61K 8/19, A61K 8/21, A61K 8/24, A61K 8/64, A61K 8/65, A61Q 11/00

(54) **KOMPOSITMATERIALIEN AUS CALCIUMVERBINDUNGEN UND PROTEINKOMPONENTEN**
COMPOSITE MATERIALS COMPRISED OF CALCIUM COMPOUNDS AND PROTEIN CONSTITUENTS
MATERIAUX COMPOSITES CONSTITUES DE COMPOSES DE CALCIUM ET DE COMPOSANTES PROTEIQUES

(30) Priorität: 02.07.1999 DE 19930335
(43) Veröffentlichungstag der Anmeldung: 27.03.2002
(73) Patentinhaber: Henkel Kommanditgesellschaft auf Aktien, 40589 Düsseldorf (DE)
(72) Erfinder: KROPF, Christian, D-40597 Düsseldorf (DE); DOLHAINE, Hans, D-41352 Glehn (DE); ROTH, Marcel, D-40591 Düsseldorf (DE); BRÜNINGHAUS, Ulrike, D-40789 Monheim (DE); WEISS, Albrecht, D-40764 Langenfeld (DE); SCHÖRKEN, Ulrich, D-42799 Leichlingen (DE); KINTRUP, Lothar, D-40595 Düsseldorf (DE); PASTURA, Amerigo, D-58453 Witten (DE); WÜLKNITZ, Peter, D-42799 Leichlingen (DE); KNIEP, Rüdiger, D-40764 Langenfeld (DE); ESCHEN, Burkhard, D-40599 Düsseldorf (DE); MEINDERS, Michael, D-47800 Krefeld (DE); LASKA, Hans, D-40627 Düsseldorf (DE); MÜLLNER, Stefan, D-40764 Langenfeld (DE)
(86) Internationale Anmeldenummer: PCT/EP2000/005813
(87) Internationale Veröffentlichungsnummer: WO 2001/001930

(56) Entgegenhaltungen:
- EP-A- 0 786 245
- WO-A-00/03747
- US-A- 5 320 844
- US-A- 5 514 210
- US-A- 5 783 217
- US-A- 6 013 591
- DATABASE CHEMICAL ABSTRACTS [Online] Database accession no. 1998:56045 XP002156976 & JP 10 017449 A (SANGI CO) 20. Januar 1998 (1998-01-20)

## Beschreibung

Die Erfindung betrifft Kompositmaterialien aus nanopartikulären schwer wasserlöslichen Calciumsalzen und Proteinkomponentenen, die sich aufgrund ihrer Zusammensetzung und Feinstruktur besonders zur Förderung der Wiederherstellung von Knochen und Zahnschmelz eignen.

Phosphatsalze des Calciums werden seit langem sowohl als Abrasivkomponenten als auch zur Förderung der Remineralisierung des Zahnschmelzes den Rezepturen von Zahnreinigungsmitteln und Zahnpflegemitteln zugesetzt. Dies gilt insbesondere für Hydroxylapatit und Fluorapatit sowie für amorphe Calciumphosphate und für Brushit (Dicalciumphosphat-dihydrat). Auch Calciumfluorid ist als Bestandteil von Zahnreinigungsmitteln und als Komponente zur Festigung des Zahnschmelzes und zur Kariesprophylaxe mehrfach beschrieben worden.

Die Verfügbarkeit von Calcium-Verbindungen für die erwünschte Remineralisierung hängt ganz entscheidend von der Teilchengröße dieser in Wasser schwerlöslichen und in den Zahnpflegemitteln dispergierten Komponenten ab. Man hat daher vorgeschlagen, diese schwerlöslichen Calciumsalze in feinster Verteilung einzusetzen.

Der Zahnschmelz sowie das Stützgewebe der Knochen bestehen überwiegend aus dem Mineral Hydroxylapatit. Im biologischen Entstehungsprozeß lagert sich Hydroxylapatit in geordneter Weise an die Proteinmatrix im Knochen oder Zahn an, die überwiegend aus Kollagen besteht. Die Ausbildung der harten und belastungsfähigen mineralischen Strukturen wird dabei durch die sogenannten Matrixproteine gesteuert, welche neben Kollagen durch weitere Proteine gebildet werden, die sich an das Kollagen anlagern und so einen strukturierten Mineralisierungsprozeß, der auch als Biomineralisation bezeichnet wird, bewirken.

Bei der Wiederherstellung von Knochenmaterial spielen sogenannte Knochenersatzmittel, welche den natürlichen Biomineralisationsprozeß fördern, eine wichtige Rolle. Derartige Mittel werden auch benötigt zur Beschichtung von Implantaten, um stoffschlüssige Verbindungen zwischen Knochen und Implantat zu erreichen, mit denen auch Zugkräfte übertragen werden können. Von besonderer Bedeutung sind hier Beschichtung mit einer hohen Bioaktivität, die zu einer wirksamen Verbundosteogenese führen. Nach dem Stand der Technik, wie ihn z. B. G. Willmann in Mat.-wiss. u. Werkstofftech. 30 (1999), 317 beschreibt, wird in der Regel Hydroxylapatit auf Implantate aufgebracht. Nachteilig an dieser Vorgehensweise ist neben der oft unzureichenden Beschleunigung des Biomineralisationsprozesses das Abplatzen der Hydroxylapatit-Schichten und ihre unbefriedigende chemische Stabilität.

Für bestimmte Anwendungen werden flüssig injizierbare Knochenersatzmaterialien benötigt. Hier ist eine besonders geringe Teilchengröße erforderlich, die bei den herkömmlichen Knochenersatzmitteln jedoch nicht in befriedigender Weise erzielt werden kann.

Unter den Knochenersatzmitteln sind Komposite aus Hydroxylapatit und Kollagen von besonderem Interesse, da sie die Zusammensetzung des natürlichen Knochens nachahmen. Eine ähnliche Situation herrscht bei der Wiederherstellung von Zahnmaterial, das zu etwa 95 % aus Hydroxylapatit besteht.

Kompositmaterialien der beschriebenen Art sind auf syntetischem Weg zugänglich, wie z. B. von B. Flautre et al. in J. Mater. Sci.: Mater. In Medicine 7 (1996), 63 beschrieben. Jedoch liegt in diesen Kompositen die Komgröße der Calciumsalze oberhalb von 1000 nm, was zu groß ist, um eine befriedigende biologische Wirkung als Remineralisierungsmittel zu erzielen.

Demgegenüber beschreibt R. Z. Wang et al., J. Mater. Sci. Lett. 14 (1995), 490 ein Herstellungsverfahren für ein Kompositmaterial aus Hydroxylapatit und Kollagen, in welchem Hydroxylapatit mit einer Partikelgröße im Bereich von 2 bis 10 nm in gleichmäßig verteilter Form auf der Kollagenmatrix abgeschieden wird. Das Kompositmaterial soll gegenüber anderen aus dem Stand der Technik bekannten Hydroxylapatit-Kollagen-Kompositen aufgrund der Feinteiligkeit des Hydroxylapatits eine bessere biologische Wirksamkeit haben. Wie im folgenden beschrieben, erfüllt jedoch auch das von R. Z. Wang et al. beschriebene Kompositmaterial nicht ausreichend das Bedürfnis nach Kompositmaterialien, welche die Zusammensetzung und die Mikrostruktur natürlichen Knochen- und Zahnmaterials nachahmen und in voll befriedigender Weise zur Remineralisation dieser natürlichen Materialien geeignet sind.

Aus dem Stand der Technik bekannte proteinhaltige Kompositmaterialien enthalten Proteine tierischer Herkunft, insbesondere aus bovinem Material gewonnene. Besonders in der Kosmetik besteht jedoch seit einigen Jahren ein zunehmender Wunsch nach Produkten, die gänzlich frei von Inhaltsstoffen tierischen Ursprungs sind. Es besteht daher ein Bedürfnis auch nach solchen Kompositmaterialien, welche keine Proteinkomponenten tierischen Ursprungs enthalten.

Ein weiterer Nachteil von aus dem Stand der Technik bekannten proteinhaltigen Kompositmaterialien besteht in ihrer oft aufwendigen Herstellung. So muß beispielsweise bei der Herstellung des bei R. Z. Wang et al. beschriebenen Komposits aus Hydroxylapatit und Kollagen unlösliches Kollagen gehandhabt und in sehr großen Lösungsmittelmengen verteilt werden, was technisch aufwendig ist. Dieses Verfahren wirft zusätzlich Probleme hinsichtlich der Entsorgung der bei der Herstellung anfallenden Abwässer auf.

Weiterhin weisen die aus dem Stand der Technik bekannten proteinhaltigen Kompositmaterialien, beispielsweise bedingt durch ihren Gehalt an unlöslichen und/oder hochmolekularen Proteinkomponenten, eine ungünstige Dispergierbarkeit auf und sind in die für ihre gewerbliche Anwendung erforderlichen Formulierungen schlecht einarbeitbar oder weisen eine unbefriedigende Dispersionsstabilität in den zum Gebrauch kommenden Zubereitungen auf.

Gegenstand von US 5,514,210 ist ein Kompositmaterial aus Apatit und einer organischen Komponente, das als Knochenersatzstoff verwendet wird. Das Kompositmaterial wurde jedoch bei der Herstellung gepresst und besitzt daher keine geeignete Morphologie, um die Remineralisation von Zähne und Knochen zu fördern.

US 5,320,844 beschreibt ebenfalls ein Knochenersatzstoff, welcher aus einer Calcium- Phosphor-Komponente und Kollagen besteht. Dieses Kompositmaterial weist jedoch keine Morphologie auf, die die Remineralisation von Knochen und Zähnen unterstützen bzw. fördern kann.

US 5,783,217 beschreibt lediglich niedrig kristalline Calcium-Phosphat Apatite, ohne einen organischen Träger. Das Fehlen des organischen Trägers wirkt sich nachteilig auf die Einbaugeschwindigkeit von Calcium in Knochen und Zähne aus und würde sich somit nicht für die Remineralisation eignen.

In JP10017449 wird ein Apatit mit einer Partikelgröße zwischen 1,0 und 5,0 µm angeführt. Nachteilig ist, dass durch die Größe der Kristallite die Aufnahme der schwerlöslichen Cacliumkomponente in Zähnen und Knochen erschwert wird.

Es wurde nun gefunden, daß bestimmte Kompositmaterialien zur Überwindung von vorstehend genannten Nachteilen des Stands der Technik geeignet sind.

Gegenstand der Erfindung sind Kompositmaterialien umfassend
a) in Wasser schwerlösliche Calciumsalze, ausgewählt aus Phosphaten, Fluoriden und Fluorophosphaten, die wahlweise zusätzlich Hydroxyl- und/oder Carbonat-Gruppen enthalten können, wobei die Calciumsalze in Form von nanopartikulären Primärteilchen mit einem mittleren Teilchendurchmesser im Bereich von 10 bis 150 nm vorliegen, und
b) Proteinkomponenten, ausgewählt aus Proteinen, Proteinhydrolysaten und Proteinhydrolysat-Derivate
   dadurch gekennzeichnet, dass die Calciumsalze in Form von stäbchenförmigen Primärteilchen vorliegen mit einer Dicke im Bereich von 2 bis 50 nm und einer Länge im Bereich von 10 bis 150 nm.

Unter Kompositmaterialien werden Verbundstoffe verstanden, welche die unter a) und b) genannten Komponenten umfassen und mikroskopisch heterogene, makroskopisch aber homogen erscheinende Aggregate darstellen, und in welchen die Primärpartikel der Calciumsalze an das Gerüst der Proteinkomponente assoziiert vorliegen Der Anteil der Proteinkomponenten in den Kompositmaterialien liegt zwischen 0,1 und 60 Gew.-%, bevorzugt jedoch zwischen 0,5 und 10 Gew.-%, bezogen auf das Gesamtgewicht der Kompositmaterialien.

Unter Primärteilchen werden die Kristallite, d. h. die Einzelkristalle der genannten Calciumsalze verstanden. Als Teilchendurchmesser soll hier der Durchmesser der Teilchen in Richtung ihrer größten Längenausdehnung verstanden werden. Unter dem mittleren Teilchendurchmesser ist ein über die Gesamtmenge des Komposits gemittelter Wert zu verstehen. Die Bestimmung der Teilchendurchmesser kann durch den Fachmann geläufige Methoden bestimmt werden, beispielsweise durch die Methode der Transmissionselektronenmikroskopie (TEM).

Der mittlere Teilchendurchmesser der nanopartikulären Primärteilchen liegt im Bereich von 10 bis 150 nm. Die Primärteilche liegen als stäbchenförmige Partikel mit einer Dicke im Bereich von 2 bis 50 nm und einer Länge im Bereich von 10 bis 150 nm von. Unter Dicke ist hier der kleinste Durchmesser der Stäbchen zu verstehen, unter Länge ihr größter Durchmesser.

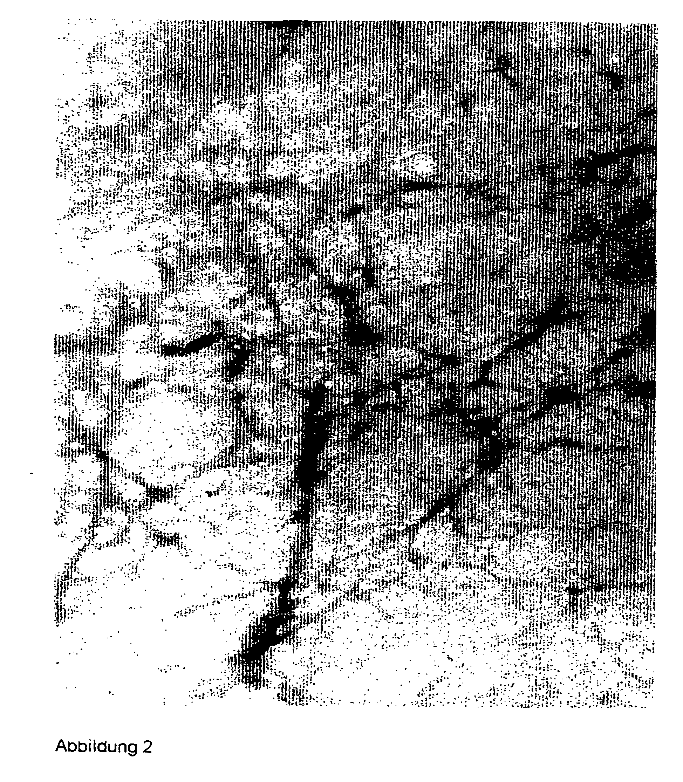

Die räumliche Struktur der erfindungsgemäßen Kompositmaterialien aus einer Proteinkomponente sowie den schwerlöslichen nanopartikulären Calciumsalzen wird am Beispiel der in Abbindung 1 dargetellten TEM-Aufnahme eines Kompositmaterials aus Hydroxylapatit und Gelatine Typ A deutlich (200.000-fache Vergrößerung; 1 cm in der Abbildung entspricht 40 nm). Der hochmolekularen Proteinkomponente, die eine im wesentlichen durch ihre Aminosäuresequenz bestimmte dreidimensionale Struktur einnimmt, sind die stäbchenförmigen Nanopartikel aus Hydroxylapatit aufgelagert, die Nanopartikel bilden also gewissermaßen die räumliche Struktur der Proteinkomponente ab. Dies wird deutlich anhand von Abbildung 2, welche eine TEM-Aufnahme des Gelatine Typ A - Gerüsts des gleichen Kompositmaterials nach Herauslösen des Hydroxylapatits mittels einer Lösung von Ethylendiamintetraacetat zeigt (56.000-fache Vergrößerung; 1,1 cm in der Abbildung entspricht 200 nm). Die Art und Weise der Anlagerung der anorganischen Partikel an das Grundgerüst der Proteinkomponente wird durch die Primärstruktur (Aminosäuresequenz) sowie je nach der Natur der Proteinkomponente ihrer Sekundär-, Tertiär- und Quartärstruktur bestimmt. Überraschenderweise wurde gefunden, daß die räumliche Verteilung und der quantitative Umfang der Anlagerung der anorganischen Nanopartikel an die Proteinkomponente durch die Art und Menge der in der Proteinkomponente vorliegenden Aminosäuren und damit durch die Auswahl der Proteinkomponenten beeinflußt werden kann. So kann beispielsweise durch die Auswahl von Proteinkomponenten, die reich an den Aminosäuren, Asparaginsäure, Glutaminsäure oder Cystein sind, eine besonders hohe Beladung mit dem schwerlöslichen Calciumsalz erreicht werden. Je nach der räumlichen Verteilung dieser Aminosäuren im Proteingerüst kann darüber hinaus eine räumlich in bestimmter Weise strukturierte Beladung der Proteinkomponente mit dem schwerlöslichen Calciumsalz erreicht werden.

Die erfindungsgemäßen Kompositmaterialien sind also strukturierte Kompositmaterialien im Gegensatz zu dem bei R. Z. Wang et al. beschriebenen Komposit aus Hydroxylapatit und Kollagen, in welchem gleichmäßig verteilte Hydroxylapatit-Nanopartikel vorliegen. Ein weiterer wesentlicher Unterschied zwischen dem Gegenstand der vorliegenden Erfindung und dem Stand der Technik besteht in der Größe und Morphologie der anorganischen Komponente. Die in dem von R. Z. Wang et al. beschriebenen Hydroxylapatit-Kollagen-Komposit vorliegenden Hydroxylapatit-Teilchen haben eine Größe von 2-10 nm. Hydroxylapatit-Partikel in diesem Größenbereich sind dem Bereich der amorphen oder teilweise röntgenamorphen Stoffe zuzurechnen.

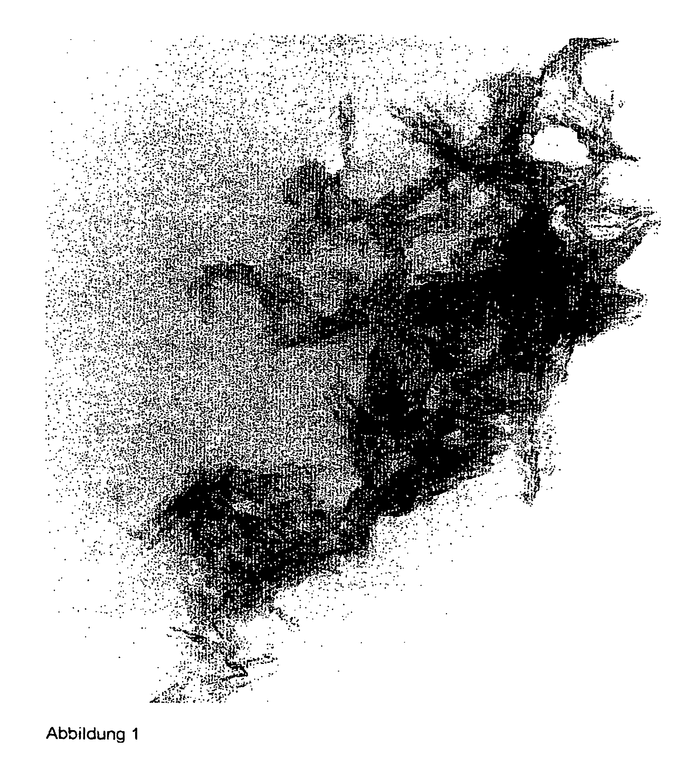

Überraschenderweise gelang es mit der vorliegenden Erfindung, Kompositmaterialien mit kristallinen anorganischen Nanopartikeln zu erzeugen, in welchen die Nanopartikel eine mikroskopisch deutlich erkennbare kristalline Morphologie aufweisen. Abbildung 1 zeigt die stäbchenförmige Struktur der anorganischen Nanopartikel. Es wurde weiterhin gefunden, daß die erfindungsgemäßen strukturierten Kompositmaterialien im Gegensatz zum Stand der Technik zu einen besonders effektiven Biomineralisationsprozeß führen. Es wird angenommen, daß dies mit der Mikrostruktur des Kompositmaterials und insbesondere der Größe und Morphologie der Calciumsalz-Kristalle zusammenhängt. So wird angenommen, daß die Längsachse der Calciumsalz-Nanopartikel eine Vorzugsrichtung für das weitere Kristallwachstum während der Biomineralisation darstellt.

Als in Wasser schwerlöslich sollen solche Salze verstanden werden, die bei 20 °C zu weniger als 1 g/l löslich sind. Bevorzugt geeignete Calciumsalze sind Calciumhydroxyphosphat (Ca₅[OH(PO₄)₃]) bzw. Hydroxylapatit, Calciumfluorphosphat (Ca₅[F(PO₄)₃]) bzw. Fluorapatit, Fluor-dotierter Hydroxylapatit der allgemeinen Zusammensetzung Ca₅(PO₄)₃(OH,F) und Calciumfluorid (Ca F₂) bzw. Fluorit (Flußspat).

Als Calciumsalz kann in den erfindungsgemäßen Kompositmaterialien eines oder auch mehrere Salze im Gemisch, ausgewählt aus der Gruppe von Phosphaten, Fluoriden und Fluorophosphaten, die wahlweise zusätzlich Hydroxyl- und/oder Carbonat-Gruppen enthalten können, im Gemisch enthalten sein.

Als Proteine kommen im Rahmen der vorliegenden Erfindung grundsätzlich alle Proteine unabhängig von ihrem Ursprung oder ihrer Herstellung in Betracht. Beispiele für Proteine tierischen Ursprungs sind Keratin, Elastin, Kollagen, Fibroin, Albumin, Casein, Molkeprotein, Plazentaprotein. Erfindungsgemäß bevorzugt aus diesen sind Kollagen, Keratin, Casein, Molkeprotein, Proteine pflanzlichen Ursprungs wie beispielsweise Weizen- und Weizenkeimprotein, Reisprotein, Sojaprotein, Haferprotein, Erbsenprotein, Kartoffelprotein, Mandelprotein und Hefeprotein können erfindungsgemäß ebenfalls bevorzugt sein.

Unter Proteinhydrolysaten sind im Rahmen der vorliegenden Erfindung Abbauprodukte von Proteinen wie beispielsweise Kollagen, Elastin, Casein, Keratin, Mandel-, Kartoffel-, Weizen-, Reis- und Sojaprotein zu verstehen, die durch saure, alkalische und / oder enzymatische Hydrolyse der Proteine selbst oder ihrer Abbauprodukte wie beispielsweise Gelatine erhalten werden. Für den enzymatischen Abbau sind alle hydrolytisch wirkenden Enzyme geeignet, wie z. B. alkalische Proteasen. Weitere geeignete Enzyme sowie enzymatische Hydrolyseverfahren sind beispielsweise beschrieben in K. Drauz und H. Waldmann, Enzyme Catalysis in Organic Synthesis, VCH-Verlag, Weinheim 1975. Bei dem Abbau werden die Proteine in kleinere Untereinheiten gespalten, wobei der Abbau über die Stufen der Polypeptide über die Oligopeptide bis hin zu den einzelnen Aminosäuren gehen kann. Zu den wenig abgebauten Proteinhydrolysaten zählt beispielsweise die im Rahmen der vorliegenden Erfindung bevorzugte Gelatine, welche Molmassen im Bereich von 15000 bis 250000 D aufweisen kann. Gelatine ist ein Polypeptid, das vornehmlich durch Hydrolyse von Kollagen unter sauren (Gelatine Typ A) oder alkalischen (Gelatine Typ B) Bedingungen gewonnen wird. Die Gelstärke der Gelatine ist proportional zu ihrem Molekulargewicht, d. h., eine stärker hydrolysierte Gelatine ergibt eine niedriger viskose Lösung. Die Gelstärke der Gelatine wird in Bloom-Zahlen angegeben. Bei der enzymatischen Spaltung der Gelatine wird die Polymergröße stark erniedrigt, was zu sehr niedrigen Bloom-Zahlen führt.

Weiterhin sind im Rahmen der vorliegenden Erfindung als Proteinhydrolysate bevorzugt die in der Kosmetik gebräuchlichen Proteinhydrolysate mit einem durchschnittlichen Molekulargewicht im Bereich von 600 bis 4000, besonders bevorzugt von 2000 bis 3500. Übersichten zu Herstellung und Verwendung von Proteinhydrolysaten sind beispielsweise von G. Schuster und A. Domsch in Seifen Öle Fette Wachse **108**, (1982) 177 bzw. Cosm.Toil. **99**, (1984) 63, von H. W. Steisslinger in Parf.Kosm. **72**, (1991) 556 und F. Aurich et al. in Tens.Surf.Det. **29**, (1992) 389 erschienen. Vorzugsweise werden erfindungsgemäß Proteinhydrolysate aus Kollagen, Keratin, Casein sowie pflanzlichen Proteinen eingesetzt, beispielsweise solche auf Basis von Weizengluten oder Reisprotein, deren Herstellung in den beiden Deutschen Patentschriften DE 19502167 C1 und DE 19502168 C1 (Henkel) beschrieben wird.

Unter Proteinhydrolysat-Derivaten sind im Rahmen der vorliegenden Erfindung chemisch und / oder chemoenzymatisch modifizierte Proteinhydrolysate zu verstehen wie beispielsweise die unter den INCI-Bezeichnungen Sodium Cocoyl Hydrolyzed Wheat Protein, Laurdimonium Hydroxypropyl Hydrolyzed Wheat Protein, Potassium Cocoyl Hydrolyzed Collagen, Potassium Undecylenoyl Hydrolyzed Collagen und Laurdimonium Hydroxypropyl Hydrolyzed Collagen bekannten Verbindungen. Vorzugsweise werden erfindungsgemäß Derivate aus Proteinhydrolysaten des Kollagens, Keratins und Caseins sowie pflanzlichen Proteinhydrolysaten eingesetzt wie z. B. Sodium Cocoyl Hydrolyzed Wheat Protein oder Laurdimonium Hydroxypropyl Hydrolyzed Wheat Protein.

Weitere Beispiele für Proteinhydrolysate und Proteinhydrolysat-Derivate, die unter den Rahmen der vorliegenden Erfindung fallen, sind beschrieben in CTFA 1997 International Buyers' Guide, John A. Wenninger et al. (Ed.), The Cosmetic, Toiletry, and Fragrance Association, Washington DC 1997, 686-688.

Die Proteinkomponente kann in jedem der erfindungsgemäßen Kompositmaterialien durch einen oder mehrere Stoffe ausgewählt aus der Gruppe von Proteinen, Proteinhydrolysaten und Proteinhydrolysat-Derivaten gebildet werden.

Als Proteinkomponenten bevorzugt sind alle strukturbildenden Proteine, Proteinhydrolysate und Proteinhydrolysat-Derivate, worunter solche Proteinkomponenten zu verstehen sind, die aufgrund ihrer chemischen Konstitution bestimmte dreidimensionale räumliche Strukturen ausbilden, die dem Fachmann aus der Proteinchemie unter den Begriffen Sekundär-, Tertiär- oder auch Quartärstruktur geläufig sind.

In einer weiteren Ausführungsform der Erfindung können die in den Kompositmaterialien vorliegenden nanopartikulären Calciumsalz-Primärteilchen von einem oder mehreren Oberflächenmodifikationsmitteln umhüllt sein.

Dadurch kann beispielsweise die Herstellung von Kompositmaterialien in solchen Fällen erleichtert werden, bei welchen sich die nanopartikulären Calciumsalze schwer dispergieren lassen. Das Oberflächenmodifikationsmittel wird an die Oberfläche der Nanopartikel adsorbiert und verändert sie dergestalt, daß die Dispergierbarkeit des Calciumsalzes zunimmt und die Agglomeration der Nanopartikel verhindert wird.

Darüber hinaus kann durch eine Oberflächenmodifikation die Struktur der Kompositmaterialien sowie die Beladung der Proteinkomponente mit dem nanopartikulären Calciumsalz beeinflußt werden. Auf diese Weise ist es bei der Anwendung der Kompositmaterialien in Remineralisationsprozessen möglich, Einfluß auf den Verlauf und die Geschwindigkeit des Remineralisationsprozesses zu nehmen.

Unter Oberflächenmodifikationsmitteln sind Stoffe zu verstehen, welche an der Oberfläche der feinteiligen Partikel physikalisch anhaften, mit diesen jedoch nicht chemisch reagieren. Die einzelnen an der Oberfläche adsorbierten Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann beispielsweise auch unter den Begriffen Emulgatoren, Schutzkolloide, Netzmittel, Detergentien etc. bekannt.

Als Oberflächenmodifikationsmittel kommen beispielsweise Emulgatoren vom Typ der nichtionogenen Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- C_{12/18}-Fettsäuremono- und -diester von Anlagerungsprodukten von 1 bis 30 Mol Ethylenoxid an Glycerin;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- Alkylmono- und -oligoglycoside mit 8 bis 22 Kohlenstoffatomen im Alkylrest und deren ethoxylierte Analoga;
- Anlagerungsprodukte von 15 bis 60 Mol Ethylenoxid an Ricinusöl und/oder gehärtetes Ricinusöl;
- Polyol- und insbesondere Polyglycerinester, wie z. B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
- Anlagerungsprodukte von 2 bis 15 Mol Ethylenoxid an Ricinusöl und/oder gehärtetes Ricinusöl;
- Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z. B. Sorbit), Alkylglucoside (z. B. Methylglucosid, Butylglucosid, Lauryl-glucosid) sowie Polyglucoside (z. B. Cellulose);
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
- Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologen-gemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für anionische Emulgatoren sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate wie beispielsweise Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfo-succinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acylglutamate und Acylaspartate, Alkyloligoglucosidsulfate, Proteinfettsäurekondensate (insbesondere pflanzliche Produkte auf Weizenbasis), und Alkyl(ether)phosphate. Sofern die anionischen Tenside Polyglycoletherketten enthalten, können diese eine konventionelle, vorzugsweise jedoch eine eingeengte Homologenverteilung aufweisen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammonium-glycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder - Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampho-lytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind.

Als Oberflächenmodifikationsmittel geeignete Schutzkolloide sind z. B. natürliche wasserlösliche Polymere wie z. B. Gummi arabicum, Stärke, wasserlösliche Derivate von wasserunlösüchen polymeren Naturstoffen wie z. B. Celluloseether wie Methylcellulose, Hydroxyethylcellulose, Carboxymethylcellulose oder modifizierte Carboxymethylcellulose, Hydroxyethyl-Stärke oder Hydroxypropyl-Guar, sowie synthetische wasserlösliche Polymere, wie z. B. Polyvinylalkohol, Polyvinylpyrrolidon, Polyalkylenglycole, Polyasparaginsäure und Polyacrylate.

In der Regel werden die Oberflächenmodifikationsmittel in einer Konzentration von 0,1 bis 50, vorzugsweise jedoch 1 bis 20 Gew.-%, bezogen auf die Calciumsalze, eingesetzt.

Als Oberflächenmodifikationsmittel bevorzugt geeignet sind vor allem die nichtionischen Tenside in einer Menge von 1 bis 20 Gew.-%, bezogen auf das Gewicht des Calciumsalzes. Als besonders wirksam haben sich die nichtionischen Tenside vom Typ der Alkyl-C8-C16-(oligo)-glucoside und der Ethoxylate des gehärteten Rizinusöls erwiesen. Die erfindungsgemäßen Kompositmaterialien werden durch Fällungsreaktionen aus wässrigen Lösungen wasserlöslicher Calciumsalze und wässrigen Lösungen wasserlöslicher Phosphat- und / oder Fluoridsalze hergestellt werden, wobei die Fällung in Gegenwart von Proteinkomponenten durchgeführt wird.Dies erfolgt vorzugsweise in der Weise, daß die Proteinkomponenten in reiner, gelöster oder kolloidaler Form der alkalischen wäßrigen Phosphat- und / oder Fluorid-Salzlösung oder der alkalischen Lösung des Calciumsalzes vor der Fällungsreaktion beigefügt werden. Alternativ können die Proteinkomponenten in reiner, gelöster oder kolloidaler Form vorgelegt und anschließend nacheinander in beliebiger Reihenfolge oder gleichzeitig mit der alkalischen Calcium-Salzlösung sowie der alkalischen Phosphat- und / oder Fluorid-Salzlösung versetzt werden.

Bei den erfindungsgemäßen Herstellverfahren kann die Zusammenfügung der einzelnen Komponenten grundsätzlich in allen möglichen Reihenfolgen erfolgen. Als Alkalisierungsmittel wird bevorzugt Ammoniak verwendet.

Eine weitere erfindungsgemäße Variante des Herstellverfahrens besteht darin, daß man die Fällung aus einer sauren Lösung eines wasserlöslichen Calciumsalzes zusammen mit einer stöchiometrischen Menge eines wasserlöslichen Phosphat- und / oder Fluoridsalzes oder aus einer sauren Lösung von Hydroxylapatit mit einem pH-Wert unterhalb von 5, bevorzugt bei einem pH-Wert unterhalb von 3, durch Anheben des pH-Werts mit wäßrigem Alkali oder Ammoniak in Gegenwart der Proteinkomponenten durchführt.

Eine weitere Verfahrensvariante besteht darin, daß man nanopartikuläre Calciumsalze in reiner oder dispergierter Form oder durch Fällungsreaktionen aus wässrigen Lösungen wasserlöslicher Calciumsalze und wässrigen Lösungen wasserlöslicher Phosphat- und / oder Fluoridsalze hergestellte Dispersionen nanopartikulärer Calciumsalze mit den Proteinkomponenten, letztere bevorzugt in gelöster oder dispergierter Form, versetzt, wobei bei der Zugabe eine beliebige Reihenfolge gewählt werden kann.

Bevorzugt wird die Lösung oder Dispersion der Proteinkomponente vorgelegt und eine Dispersion des nanopartikulären Calciumsalzes zugefügt.

Bei allen Verfahren, im Verlauf derer eine Fällung von Apatit stattfindet, empfiehlt es sich, den pH-Wert unterhalb von 5, vorzugsweise unterhalb von 3 zu halten. Bei allen genannten Herstellverfahren kann die entstehende Dispersion des Kompositmaterials nach Bedarf durch dem Fachmann bekannte Verfahren wie z. B. Filtration oder Zentrifugation vom Lösungsmittel und den übrigen Bestandteilen des Reaktionsgemischs abgetrennt und durch anschließende Trocknung, z. B. durch Gefriertrocknung, in lösungsmittelfreier Form isoliert werden.

Als Lösungsmittel wird bei allen Herstellungsprozessen bevorzugt Wasser verwendet, jedoch können in einzelnen Schritten der Herstellung auch organische Lösungsmittel wie z. B. Alkohole mit 1 bis 4 C-Atomen oder Glycerin verwendet werden.

Die Herstellung der erfindungsgemäßen Kompositmaterialien, in welchen die Primärpartikel der Calciumsalze oberflächenmodifiziert sind, kann nach analogen Fällungsverfahren wie vorstehend beschrieben erfolgen, wobei jedoch die Fällung der nanopartikulären Calciumsalze oder der Kompositmaterialien in Gegenwart eines oder mehrerer Oberflächenmodifikationsmittel erfolgt.

Bevorzugt wird zunächst durch eine Fällungsreaktion zwischen wäßrigen Lösungen von Calciumsalzen und wäßrigen Lösungen von Phosphat- und / oder Fluoridsalzen in Gegenwart der Oberflächenmodifikationsmittel die oberflächenmodifizierten nanopartikulären Calciumsalze erzeugt. Diese können anschließend von Begleitprodukten des Reaktionsgemischs gereinigt werden, z. B. durch Einengen unter reduziertem Druck und anschließende Dialyse. Durch Abziehen des Lösungsmittels kann zusätzlich eine Dispersion des oberflächenmodifizierten Calciumsalzes mit einem Feststoffanteil nach Wunsch hergestellt werden. Anschließend wird durch Zugabe der Proteinkomponenten in reiner, gelöster oder kolloidaler Form, wobei wiederum die Reihenfolge der Zugabe unkritisch ist, und erforderlichenfalls Nachreaktion bei erhöhter Temperatur, bevorzugt im Bereich zwischen 50 und 100 °C und für eine Dauer von 1 bis 100 Minuten, das Kompositmaterial aus oberflächenbeschichtetem Calciumsalz und Proteinkomponenten gebildet.

Zur Herstellung von Dispersionen oberflächenmodifizierter Calciumsalze können weitere Verfahren herangezogen werden, wie die in der deutschen Anmeldung DE 19858662.0 beschriebenen.

Die erfindungsgemäßen Kompositmaterialien, insbesondere die von Hydroxylapatit, Fluorapatit und Calciumfluorid, eignen sich als remineralisierende Komponente zur Herstellung von Zusammensetzungen zur Reinigung und/oder Pflege der Zähne. Durch die strukturierte Form der Komposite und die Partikelgröße der darin enthaltenen Calciumverbindungen kann die Wirkung einer Festigung des Zahnschmelzes und des Verschlusses von Läsionen und Dentinkanälchen besonders rasch und vollständig erfolgen. Die Zusammensetzungen zur Reinigung und Pflege der Zähne können dabei beispielsweise in Form von Pasten, flüssigen Cremes, Gelen oder Mundspülungen vorliegen. Selbst in flüssigen Zubereitungen verteilen sich die erfindungsgemäßen Kompositmaterialien leicht, bleiben stabil dispergiert und neigen nicht zur Sedimentation.

Eine bevorzugte Ausführungsform sind Zahnpasten mit einem Gehalt an Kieselsäure, Poliermitteln, Feuchthaltemitteln, Bindemitteln und Aromen, die 0,1 - 10 Gew.% erfindungsgemäße Kompositmaterialien mit nanopartikulären Calciumsalzen aus der Gruppe Hydroxylapatit, Fluorapatit und Calciumfluorid enthalten.

Die Zubereitungen zur Reinigung und Pflege der Zähne können dabei die üblichen Komponenten und Hilfsmittel solcher Zusammensetzungen in den dafür üblichen Mengen enthalten. Für Zahnpasten sind dies z. B.
- Putz- und Polierkörper wie z. B. Kreide, Kieselsäuren, Aluminiumhydroxid, Aluminiumsilikate, Calciumpyrophosphat, Dicalciumphosphat, unlösliches Natriummetaphosphat oder Kunstharzpulver
- Feuchthaltemittel wie z. B. Glycerin, 1,2-Propylenglycol, Sorbit, Xylit und Polyethylenglycole
- Bindemittel und Konsistenzregler, z. B. natürliche und synthetische wasserlösliche Polymere und wasserlösliche Derivate von Naturstoffen, z. B. Celluloseether, Schichtsilikate, feinteilige Kieselsäuren (Aerogel-Kieselsäuren, pyrogene Kieselsäuren)
- Aromen, z. B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen
- Süßstoffe wie z. B. Saccharin-Natrium, Natriumcyclamat, Aspartame, Acesulfan K, Steviosid, Monellin, Glycyrrhicin, Dulcin, Lactose, Maltose oder Fructose
- Konservierungsmittel und antimikrobielle Stoffe wie z. B. p-Hydroxybenzoesäureester, Natriumsorbat, Triclosan, Hexachlorophen, Phenylsalicylsäureeter, Thymol usw.
- Pigmente wie z. B. Titandioxid oder Pigmentfarbstoffe zur Erzeugung farbiger Streifen
- Puffersubstanzen z. B. primäre, sekundäre oder tertiäre Alkaliphosphate, Citronensäure / Na-Citrat
- wundheilende und entzündungshemmende Wirkstoffe, z. B. Allantoin, Harnstoff, Azulen, Panthenol, Acetylsalicylsäure-Derivate, Pflanzenextrakte, Vitamine, z. B. Retinol oder Tocopherol.

Die erfindungsgemäßen Kompositmaterialien, insbesondere die von Hydroxylapatit und Fluorapatit, können die Biomineralisation in Knochengewebe induzieren oder fördern. Sie eignen sich daher weiterhin als biomineralisierende Komponente zur Herstellung von Zusammensetzungen zur Wiederherstellung oder Neubildung von Knochenmaterial, wie z. B. von Zusammensetzungen zur Behandlung von Knochendefekten und Knochenfakturen sowie zur Förderung des Einwachsens von Implantaten.

Zur Beschichtung von Implantaten können die erfindungsgemäßen Kompositmaterialien beispielsweise nach den dem Fachmann bekannten Standardverfahren der Tauchbeschichtung oder des Plasmaspritzens aufgebracht werden.

Zur Verwendung als injizierbare Knochenersatzmaterialien können die erfindungsgemäßen Kompositmaterialien mit geeigneten weiteren Stoffen kombiniert werden, wie z. B. Glycosaminoglycanen oder Proteinen, und mit geeigneten Lösungs- und Hilfsmitteln wie z. B. einem verdünnten wäßrigen Phosphatpuffer formuliert werden.

Die folgenden Beispiele sollen den Erfindungsgegenstand näher erläutern:

### Beispiele

**1. Herstellung von Proteinlösungen bzw. -dispersionen**
1.1 Gelatine Typ A :
   10 g Gelatine Typ A (Gelatine gewonnen durch saure Hydrolyse von Schweinehaut) wurden mit 100 ml Wasser versetzt und mittels Mikrowelle einmal aufgekocht.
1.2 Gelatine Typ A und Casein:
   10 g Gelatine Typ A wurden mit 100 ml Wasser sowie 10 ml des Überstands einer bei 20°C gesättigten und anschließend mit 5000 rpm zentrifugierten Caseinlösung versetzt und anschließend mittels Mikrowelle einmal aufgekocht.
1.3 Hydrolysat von Gelatine Typ A:
   10 g Gelatine Typ A wurden mit 100 ml Wasser sowie der alkalischen Protease Savinase (Hersteller: Novo Nordisk) in einer Einsatzkonzentration von 0,005 % Enzym-Trockensubstanz, bezogen auf die Trockensubstanz der Gelantine versetzt. Nach 20 h Rühren bei 20 °C wurde mittels Mikrowelle einmal aufgekocht.
1.4 Hydrolysat von Gelatine Typ A und Casein:
   10 g Gelatine Typ A und 1 g Casein wurden mit 100 ml H2O versetzt, über Nacht bei Raumtemperatur mit alkalischer Protease Savinase (Hersteller: Novo Nordisk) in einer Einsatzkonzentration von 0,005 % Enzym-Trockensubstanz, bezogen auf die Trockensubstanz der Proteinkomponenten hydrolysiert, dann einmal in der Mikrowelle aufgekocht und anschließend filtriert.
1.5 Gelatine Typ B :
   10 g Gelatine Typ B (Gelatine gewonnen durch alkalische Hydrolyse von Rinderhaut) wurden mit 100 ml Wasser versetzt und mittels Mikrowelle einmal aufgekocht.
1.6 Gelatine Typ B und Casein:
   10 g Gelatine Typ B wurden mit 100 ml Wasser sowie 10 ml des Überstands einer bei 20°C gesättigten und anschließend mit 5000 rpm zentrifugierten Caseinlösung versetzt und anschließend mittels Mikrowelle einmal aufgekocht.
1.7 Hydrolysat von Gelatine Typ B:
   10 g Gelatine Typ B wurden mit 100 ml Wasser sowie der alkalischen Protease Savinase (Hersteller: Novo Nordisk) in einer Einsatzkonzentration von 0,005 % Enzym-Trockensubstanz bezogen auf die Trockensubstanz der Gelatine versetzt. Nach 20 h Rühren bei 20 °C wurde mittels Mikrowelle einmal aufgekocht.
1.8 Hydrolysat von Gelatine Typ B und Casein:
   10 g Gelatine Typ B und 1 g Casein wurden mit 100 ml H2O versetzt, über Nacht bei Raumtemperatur mit alkalischer Protease Savinase (Hersteller: Novo Nordisk) in einer Einsatzkonzentration von 0,005 % Enzym-Trockensubstanz bezogen auf die Trockensubstanz der Proteinkomponenten hydrolysiert, dann einmal in der Mikrowelle aufgekocht und anschließend filtriert.

**2. Herstellung von Kompositmaterialien durch Fällungsreaktionen in Gegenwart der Proteinkomponenten**
2.1 Kompositmaterial aus Hydroxylapatit und Gelatine Typ A:
   2,21 g Calciumchlorid wurden in 137 ml vollentsalztes Wasser gelöst, auf 25 °C temperiert und mit 25 Gew.-%iger wäßriger Ammoniaklösung auf pH = 11 eingestellt. Unter starkem Rühren wurden anschließend 20 ml der im Wasserbad auf 30-40 °C erwärmten nach Beispiel 1.1 hergestellten Proteinlösung zugefügt. Im Anschluß wurde eine wäßrige Lösung von 1,58 g Diammoniumhydrogenphosphat in 26 ml vollentsalztes Wasser, welche auf 25 °C temperiert und mit Ammoniaklösung auf pH = 11 eingestellt worden war, langsam innerhalb von 1 h zugetropft. Dabei findet die Ausfällung des Kompositmaterials statt. Der pH-Wert lag zu Beginn der Zutropfzeit bei 10,4 und wurde durch Nachdosierung von Ammoniaklösung bei einem pH-Wert von ca. 10 gehalten. Nach 20 h Reaktionszeit (25°C, unter Rühren) war der pH-Wert der wäßrigen Suspension auf 9,5 abgefallen. Das ausgefallene Kompositmaterial wurde bei 5000 rpm abzentrifugiert mit ca. 30 - 40°C warmem vollentsalztem Wasser gewaschen und gefriergetrocknet. Man erhielt 2,2 g Kompositmaterial, dessen Elementaranalyse einen Kohlenstoffgehalt von 2,3 % ergab; dies entspricht einem Gehalt an Proteinmaterial von 5,6 Gew.-%, bezogen auf die Gesamtmenge des Kompositmaterials.
2.2-2.8 Kompositmaterialien aus Hydroxylapatit und weiteren Proteinkomponenten:
   In analoger Weise wie unter Beispiel 2.1 beschrieben erhielt man Kompositmaterialien aus Hydroxylapatit sowie den unter 1.2 bis 1.8 beschriebenen Proteinkomponenten.

**3. Herstellung von Kompositmaterialien durch Einarbeitung von Dispersionen oberflächenmodifizierter Calciumsalze in Proteinkomponenten**
3.1 Kompositmaterial aus Hydroxylapatit und Gelatine Bloom 300:
   Zunächst wurden getrennt die Lösungen A und B hergestellt.
   Lösung A:
      25.4 g Calciumnitrat-Tetrahydrat und 8.50 g Diammoniumhydrogenphosphat wurden jeweils in 100 g entionisiertem Wasser gelöst. Beide Lösungen wurden unter Ausbildung eines weißen Niederschlags zusammengegeben. Nach Zugabe von 10 ml 37 Gew.-%iger HCl erhielt man eine klare Lösung.
   Lösung B:
      200 ml entionisiertes Wasser, 200 ml 25 Gew.-%ige wäßrige Ammoniaklösung sowie 20 g Plantacare® 1200 wurden zusammengegeben und auf 0°C im Eisbad abgekühlt. Unter Ausbildung eines Hydroxylapatit-Niederschlages gab man unter starkem Rühren Lösung A zu Lösung B. Nach Abziehen überschüssigen Ammoniaks wurde die Dispersion mittels Dialyse gereinigt. Am Rotationsverdampfer engte man die Dispersion durch Bestimmung der abgeschiedenen Wassermenge anschließend soweit ein, daß der Feststoffanteil in der Dispersion, berechnet als Hydroxylapatit, 7.5 Gew.-% betrug.
      Diese Dispersion wurde bei Raumtemperatur zu 100 ml g einer analog Beispiel 1.1 hergestellten 10 Gew.-%igen wässrigen Lösung von Gelatine Bloom 300 (Hersteller: Fluka) zugegeben, dann auf 80°C erwärmt und bei dieser Temperatur 5 Minuten gerührt. Anschließend ließ man die Masse unter Ausbildung des Kompositmaterials bei Raumtemperatur erstarren.

**4. Zahncremes mit Calciumsalz-Kompositmaterialien**

| | | |
|---|---|---|
| **Rezeptur-Beispiele** | **4.1** | **4.2** |
| Sident® 8 | 10,0 Gew.-% | 10,0 Gew.-% |
| Sident® 22S | 7,0 Gew.-% | 7,0 Gew.-% |
| Sipernat® 320DS | 0,8 Gew.-% | 0,8 Gew.-% |
| Kompositmaterial gemäß Beispiel 2.1 | 5,0 Gew.-% | - |
| Kompositmaterial gemäß Beispiel 3.1 | - | 5,0 Gew.-% |
| Polywachs® 1550 | 2,0 Gew.-% | 2,0 Gew.-% |
| Texapon® K 1296 | 1,5 Gew.-% | 1,5 Gew.-% |
| Titandioxid | 1,0 Gew.-% | 1,0 Gew.-% |
| Cekol® 500 T | 1,0 Gew.-% | 1,0 Gew.-% |
| Na-Fluorid | 0,33 Gew.-% | 0,33 Gew.-% |
| Na-Benzoat | 0,25 Gew.-% | 0,25 Gew.-% |
| Aroma | 1,0 Gew.-% | 1,0 Gew.-% |
| Tagat® S | 0,2 Gew.-% | - |
| Na-Saccharinat | 0,15 Gew.-% | 0,15 Gew.-% |
| Tri-Natriumphosphat | 0,10 Gew.-% | 0,10 Gew.-% |
| Sorbit (70 %ig in Wasser) | 31,0 Gew.-% | 31,0 Gew.-% |
| Wasser | ad 100 Gew.-% | ad 100 Gew.-% |

Es wurden folgende Handelsprodukte verwendet:

| | |
|---|---|
| Plantacare®1200: | C₁₂-C₁₆-Alkylglycosid |
| | ca. 50 Gew.-% in Wasser |
| | Hersteller: HENKEL KgaA |
| | |
| Sident®8: | Synth. amorph. Kieselsäure, BET 60 m²/g |
| | Stampfdichte: 350 g/l |
| | Hersteller: DEGUSSA |
| | |
| Sident®22 S: | Hydrogelkieselsäure, BET 140 m²/g |
| | Stampfdichte: 100 g/l |
| | Hersteller: DEGUSSA |
| | |
| Polywachs® 1550: | Polyethylenglycol, MG : 1550 |
| | Erweichungspunkt 45 - 50°C |
| | Hersteller: RWE / DEA |
| | |
| Texapon®K 1296: | Natrium-Laurylsulfat-Pulver |
| | Hersteller: HENKEL KGaA |
| | |
| Cekol®500 T: | Natrium-Carboxymethylcellulose |
| | Viskosität (2 %ig in Wasser, Brookfield LVF 20°C) : 350 - 700 mPa·s |
| | Lieferant: Nordmann-Rassmann |
| | |
| Tagat® S: | Polyoxyethylen-(20)-glycerylmonostearat |
| | Hersteller: Tego Cosmetics (Goldschmidt) |

## Patentansprüche

1. Kompositmaterialien umfassend
a) in Wasser schwerlösliche Calciumsalze, ausgewählt aus Phosphaten, Fluoriden und Fluorophosphaten, die wahlweise zusätzlich Hydroxyl- und/oder Carbonat-Gruppen enthalten können, wobei die Calciumsalze in Form von nanopartikulären Primärteilchen mit einem mittleren Teilchendurchmesser im Bereich von 10 bis 150 nm vorliegen, und
b) Proteinkomponenten ausgewählt aus Proteinen, Proteinhydrolysaten und Proteinhydrolysat-Derivaten
**dadurch gekennzeichnet, dass** die Calciumsalze in Form von stäbchenförmigen Primärteilchen vorliegen mit einer Dicke im Bereich von 2 bis 50 nm und einer Länge im Bereich von 10 bis 150 nm.

2. Kompositmaterialien nach Anspruch 1, **dadurch gekennzeichnet, daß** die Proteinkomponenten ausgewählt sind aus strukturbildenden Proteinen, Proteinhydrolysaten und Proteinhydrolysat-Derivaten.

3. Kompositmaterialien nach einem der Ansprüche 1 oder 2, **dadurch gekennzeichnet, daß** die Proteinkomponenten ausgewählt sind aus Kollagen, Gelatine, Keratin, Casein, Weizenprotein, Reisprotein, Sojaprotein, Mandelprotein und deren Hydrolysaten und Hydrolysat-Derivaten.

4. Kompositmaterialien gemäß Anspruch 3, **dadurch gekennzeichnet, daß** die Proteinkomponenten ausgewählt sind aus Gelatine, Casein und deren Hydrolysaten.

5. Kompositmaterialien nach einem der Ansprüche 1 bis 4, **dadurch gekennzeichnet, daß** die als nanopartikuläre Primärpartikel vorliegenden Calciumsalze von einem oder mehreren Oberflächenmodifikationsmitteln umhüllt sind.

6. Kompositmaterialien nach einem der Ansprüche 1 bis 5, **dadurch gekennzeichnet, daß** das Calciumsalz ausgewählt ist aus der Gruppe Hydroxylapatit und Fluorapatit.

7. Kompositmaterialien nach einem der Ansprüche 1 bis 6, **dadurch gekennzeichnet, daß** der Anteil der Proteinkomponenten im Kompositmaterial zwischen 0,5 und 10 Gew.-% bezogen auf das Gesamtgewicht des Kompositmaterials beträgt.

8. Verfahren zur Herstellung von Kompositmaterialien nach einem der Ansprüche 1 bis 7 durch Fällungsreaktionen aus wäßrigen Lösungen wasserlöslicher Calciumsalze und wäßrigen Lösungen wasserlöslicher Phosphat- und / oder Fluoridsalze, **dadurch gekennzeichnet, daß** die Fällung in Gegenwart von Proteinkomponenten durchgeführt wird.

9. Verfahren zur Herstellung von Kompositmaterialien nach Anspruch 8 durch Fällung aus einer sauren Lösung eines wasserlöslichen Calciumsalzes und einer stöchiometrischen Menge eines wasserlöslichen Phosphat- und / oder Fluoridsalzes mit einem pH-Wert unterhalb von 3 durch Anheben des pH-Wertes mit wäßrigen Alkalien oder Ammoniak in Gegenwart von Proteinkomponenten.

10. Verwendung der Kompositmaterialien nach einem der Ansprüche 1 bis 7 als remineralisierende Komponente zur Herstellung einer Zusammensetzung zur Reinigung und / oder Pflege der Zähne.

11. Verwendung von Kompositmaterialien nach einem der Ansprüche 1 bis 7 als die Biomineralisation induzierende oder fördernde Komponente zur Herstellung einer Zusammensetzung für die Behandlung von Zahn- oder Knochendefekten.

12. Verwendung von Kompositmaterialien nach einem der Ansprüche 1 bis 7 zur Herstellung einer Zusammensetzung zur Festigung des Zahnschmelzes.

13. Verwendung der Kompositmaterialien nach einem der Ansprüche 1 bis 7 zur Herstellung einer Zusammensetzung zur Beschichtung von Implantaten.

14. Zahnpasten mit einem Gehalt an Kompositmaterialien nach einem der Ansprüche 1 bis 7.

15. Zusammensetzungen zur Induktion oder Förderung der Neubildung von Knochengewebe mit einem Gehalt an Kompositmaterialien nach einem der Ansprüche 1 bis 7.

## Claims

1. Composite materials comprising
a) calcium salts poorly soluble in water selected from phosphates, fluorides and fluorophosphates which, optionally, may additionally contain hydroxyl and/or carbonate groups, the calcium salts being present in the form of nanoparticulate primary particles with a mean particle diameter of 10 to 150 nm, and
b) protein components selected from proteins, protein hydrolyzates and protein hydrolyzate derivatives,
**characterized in that** the calcium salts are present in the form of rodlet-like primary particles with a thickness of 2 to 50 nm and a length of 10 to 150 nm.

2. Composite materials as claimed in claim 1, **characterized in that** the protein components are selected from structuring proteins, protein hydrolyzates and protein hydrolyzate derivatives.

3. Composite materials as claimed in claim 1 or 2, **characterized in that** the protein components are selected from collagen, gelatin, keratin, casein, wheat protein, rice protein, soya protein, almond protein and hydrolyzates and hydrolyzate derivatives thereof.

4. Composite materials as claimed in claim 3, **characterized in that** the protein components are selected from gelatin, casein and hydrolyzates thereof.

5. Composite materials as claimed in any of claims 1 to 4, **characterized in that** the calcium salts present as nanoparticulate primary particles are coated with one or more surface modifiers.

6. Composite materials as claimed in any of claims 1 to 5, **characterized in that** the calcium salts is selected from the group consisting of hydroxylapatite and fluoroapatite.

7. Composite materials as claimed in any of claims 1 to 6, **characterized in that** the percentage content of protein components in the composite material is between 0.5 and 10% by weight, based on the total weight of the composite material.

8. A process for the production of the composite materials claimed in any of claims 1 to 7 by precipitation reactions from aqueous solutions of water-soluble calcium salts and aqueous solutions of water-soluble phosphate and/or fluoride salts, **characterized in that** the precipitation is carried out in the presence of protein components.

9. A process for the production of composite materials as claimed in claim 9 by precipitation from an acidic solution of a water-soluble calcium salt and a stoichiometric quantity of a water-soluble phosphate and/or fluoride salt with a pH below 3 by raising the pH with aqueous alkalis or ammonia in the presence of protein components.

10. The use of the composite materials claimed in any of claims 1 to 7 as remineralizing components for the production of tooth cleaning and/or dental care compositions.

11. The use of the composite materials claimed in any of claims 1 to 7 as a biomineralization-inducing or -promoting component for the production of a composition for the treatment of tooth or bone defects.

12. The use of the composite materials claimed in any of claims 1 to 7 for the production of a composition for strengthening dental enamel.

13. The use of the composite materials claimed in any of claims 1 to 7 for the production of a composition for coating implants.

14. Toothpastes containing the composite materials claimed in any of claims 1 to 7.

15. Compositions for inducing or promoting the formation of new bone tissue containing the composite materials claimed in any of claims 1 to 7.

## Revendications

1. Matériaux composites comprenant
a) des sels de calcium difficilement solubles dans l'eau, choisis parmi le groupe comprenant des phosphates, des fluorures et des fluorophosphates, qui peuvent contenir en outre, de manière facultative, des groupes hydroxyle et/ou des groupes carbonate, les sels de calcium étant présents sous la forme de particules primaires nanoparticulaires dont le diamètre moyen de particule se situe dans la plage de 10 à 150 nm, et
b) des composants protéiques choisis parmi le groupe comprenant des protéines, des hydrolysats de protéines et des dérivés d'hydrolysats de protéines
**caractérisés en ce que** les sels de calcium sont présents sous la forme de particules primaires en forme de bâtonnets dont l'épaisseur se situe dans la plage de 2 à 50 nm et dont la longueur se situe dans la plage de 10 à 150 nm.

2. Matériaux composites selon la revendication 1, **caractérisés en ce que** les composants protéiques sont choisis parmi le groupe comprenant des protéines, des hydrolysats de protéines et des dérivés d'hydrolysats de protéines de type structurant.

3. Matériaux composites selon la revendication 1 ou 2, **caractérisés en ce que** les composants protéiques sont choisis parmi le groupe comprenant du collagène, de la gélatine, de la kératine, de la caséine, de la protéine de froment, de la protéine de riz, de la protéine de soja, de la protéine d'amande, ainsi que leurs hydrolysats et leurs dérivés d'hydrolysats.

4. Matériaux composites selon la revendication 3, **caractérisés en ce que** les composants protéiques sont choisis parmi le groupe comprenant la gélatine, la caséine et leurs hydrolysats.

5. Matériaux composites selon l'une quelconque des revendications 1 à 4, **caractérisés en ce que** les sels de calcium présents sous la forme de particules primaires nanoparticulaires sont enveloppés d'un ou de plusieurs agents de modification de la surface.

6. Matériaux composites selon l'une quelconque des revendications 1 à 5, **caractérisés en ce que** le sel de calcium est choisi parmi le groupe comprenant l'hydroxylapatite et la fluoroapatite.

7. Matériaux composites selon l'une quelconque des revendications 1 à 6, **caractérisés en ce que** la fraction des composants protéiques dans le matériau composite s'élève entre 0,5 et 10 % en poids, rapportés au poids total du matériau composite.

8. Procédé pour la préparation de matériaux composites selon l'une quelconque des revendications 1 à 7, via des réactions de précipitation à partir de solutions aqueuses de sels de calcium solubles dans l'eau et à partir de solutions aqueuses de sels de phosphates et/ou de fluorures solubles dans, l'eau, **caractérisé en ce qu'**on effectue la précipitation en présence de composants protéiques.

9. Procédé pour la préparation de matériaux composites selon la revendication 8, par précipitation à partir d'une solution acide d'un sel de calcium soluble dans l'eau et d'une quantité stoechiométrique d'un sel de phosphate et/ou de fluorure soluble dans l'eau possédant une valeur de pH inférieure à 3, par élévation de la valeur du pH avec des alcalis aqueux ou avec de l'ammoniaque en présence de composants protéiques.

10. Utilisation des matériaux composites selon l'une quelconque des revendications 1 à 7, à titre de composant reminéralisant pour la préparation d'une composition destinée au nettoyage et/ou au soin des dents.

11. Utilisation des matériaux composites selon l'une quelconque des revendications 1 à 7, comme composant induisant ou favorisant la biominéralisation pour la préparation d'une composition destinée au traitement de déficiences dentaires ou osseuses.

12. Utilisation des matériaux composites selon l'une quelconque des revendications 1 à 7, pour la préparation d'une composition destinée au renforcement de l'émail des dents.

13. Utilisation des matériaux composites selon l'une quelconque des revendications 1 à 7, pour la préparation d'une composition destinée au recouvrement d'implants.

14. Dentifrices possédant une teneur en matériaux composites selon l'une quelconque des revendications 1 à 7.

15. Compositions pour induire ou favoriser la reconstitution de tissu osseux, possédant une teneur en matériaux composites selon l'une quelconque des revendications 1 à 7.
